**Europäisches Patentamt**

(19) **European Patent Office**

**Office européen des brevets**

(11) Publication number : **0 429 523 B1**

# (12) EUROPEAN PATENT SPECIFICATION

(45) Date of publication of patent specification :
**12.08.92 Bulletin 92/33**

(51) Int. Cl.⁵ : **A61M 16/04**

(21) Application number : **89909721.6**

(22) Date of filing : **15.08.89**

(86) International application number :
**PCT/DK89/00195**

(87) International publication number :
**WO 90/01964 08.03.90 Gazette 90/06**

(54) ENDOTRACHEAL TUBE.

(30) Priority : **22.08.88 DK 4700/88**
**03.03.89 DK 1031/89**

(43) Date of publication of application :
**05.06.91 Bulletin 91/23**

(45) Publication of the grant of the patent :
**12.08.92 Bulletin 92/33**

(84) Designated Contracting States :
**AT BE CH DE FR GB IT LI LU NL SE**

(56) References cited :
**US-A- 4 150 676**
**US-A- 4 329 983**
**US-A- 4 589 410**
**US-A- 4 622 965**
**Derwent's abstract, No. 87-354 103/50, SU 1**
**308 332, publ. week 8750(ZAPORO**
**PUBLICHEALT (KINE))**

(73) Proprietor : **Siemssen, J. Siems**
**Hannerupgaerdsvej 42**
**DK-5230 Odense M (DK)**
Proprietor : **Siemssen, Ole**
**Lundsgade 4**
**DK-2100 Copenhagen (DK)**
Proprietor : **Siemssen, Peter A.**
**c/o Jamroth Marstalsgade 3**
**DK-2100 Copenhagen (DK)**

(72) Inventor : **Siemssen, J. Siems**
**Hannerupgaerdsvej 42**
**DK-5230 Odense M (DK)**
Inventor : **Siemssen, Ole**
**Lundsgade 4**
**DK-2100 Copenhagen (DK)**
Inventor : **Siemssen, Peter A.**
**c/o Jamroth Marstalsgade 3**
**DK-2100 Copenhagen (DK)**

(74) Representative : **Tscherning, Christian et al**
**c/o Internationalt Patent-Bureau Hoeje**
**Taastrup Boulevard 23**
**DK-2630 Taastrup (DK)**

**EP 0 429 523 B1**

Jouve, 18, rue Saint-Denis, 75001 PARIS

## Description

The invention relates to an endotracheal tube for blind, nasal intubation, and of the type in which the tube comprises a distal end, a proximal end, an inflatable cuff positioned at a relatively short distance from the distal end, said cuff having an associated channel extending longitudinally in the wall tube and intended to connect the interior of the cuff with an air supply hose provided with valve, means for altering the curvature of the tube during intubation

The traditional intubation with exposure of aditus laryngis necessitates general anaesthesia and total muscular relaxation by injection of a curare preparation, frequent venting on mask and putting on a mouth piece in upper jaw. The exposure of aditus laryngis by means of a laryngoscope may require, particularly in adult male patients, quite strong arms, a requirement which nurse anaesthetists or female anaesthesiologists are not always able to fulfil, and the intubation procedure is therefore often time-consuming and time-wasting.

The starting point of the invention is the anatomically close relation between aditus oesophagus and aditus trachea along with the fact that respiration continues spontaneously after the patient has been fully anaesthetized by an intravenous injection of a quickly acting preparation of barbituric acid. By means of a stethoscope connected with the tube it will thus be possible to locate the position of the tip of a tube inserted in respiratory and oesophageal passages. Upon cessation of the spontaneous respiration the tube tip has engaged the oesophagus orifice and upon resumption of respiration the tube tip is located on a level with aditus laryngis.

An endotracheal tube of the above mentioned type is disclosed in US patent No. 4 150 676. The above stated means of altering the curvature of the tube during intubation consist, one one hand, in a pull-wire passed through a longitudinal channel in the tube wall and which at the distal end is firmly connected with said tube wall and, on the other hand, of a ring connected with said pull wire at the proximal end of the tube. During intubation the operator may be varying the pulling force on the ring more or less alter the curvature of the tube according to the lumen in patient's nasal fossae, pharynx and aditus laryngis.

Moreover, this guiding principle with tensioning cords is also disclosed, for instance in US patent No. 3 470 876 dealing with a catheter for another purpose, the curvature of the proximal end of said catheter being altered in the same manner.

A drawback of the prior art disclosed in said US patent No. 4 150 676 is that the "blind", i.e. the non-laryngoscopic intubation implies some sort of remote control of the tube tip in the form of a forward tilting in order to engage aditus laryngis when inserting the tube, and that the tensioning on the ring provokes the curvature of the tube to change, i.e. increased curvature throughout the length of the tube. Such an alteration of the curvature of the tube which, as said, applies to the entire length of the tube means that blind intubation effected in this manner only succeeds after repeated attempts with varying bending of the patient's head and varying curvature of the tube.

Such repeated attempts further increase the risk of traumatizing the mucous membrane. The problem of adapting the curvature of the tube to comply with the anatomic configuration is also emphasized in said US reference (column 3, lines 48 to column 4, line 14) stating that the operator at varying tensioning may hold the tube in various points, distally or centrally, with a view to redistributing the curving effect. This implies the use of both hands when inserting and pulling back the tube until the distal end has been put in place at aditus laryngis.

The invention aims at eliminating said drawbacks and contemplates the provision of a tube structure allowing a considerably easier intubation which is, moreover, more gentle to the patient.

In this respect an endotracheal tube of the above mentioned type is according to the invention characterized in that the tube between the distal end and the cuff presents a smaller flexural rigidity than the remainder of the tube.

Such an endotracheal tube offers the following advantages.

With the fixed point in the tube tip circumferentially of the zone where the tube has a smaller flexural rigidity and by the tensile means an arbitrary tilting and straightening of the tube tip will be possible about a transverse axis at the zone where the tube has a smaller flexural rigidity. This provides for obtaining a selective, graduated function of the tube tip during a two-phased intubation procedure while making use of an auscultatory-tactile control. The description of the technique practised for the intubation manoeuvre implies that the patient is in supine position, the nose turning right up and the neck resting on the bed without any intermediate layer, but the tube according to the invention allows for placing the patient in side position, the so-called NATO-position, or in abdominal position.

Realizing that this is a non-visually controlled intubation and in order to ensure a procedure traumatizing the patient as little as possible, it is important that the bevelled edge of the tube tip during insertion is as close to the plane of glottis as possible. This is obtained by inserting the tube tip through nostril with its cylindrical portion against nasal septum, following which the bevelled edge of the tube tip upon penetrating pharynx lumen will be located at the same plane as glottis or very close thereto.

In accordance with an advantageous embodiment said smaller flexural rigidity may be obtained in that at least one, mainly V-shaped incision is provided in the tube wall, that a flexible bellows-shaped membrane is provided for air-, gas- and liquidtight closing of the incision, and that a point of the tube wall between the distal end and the nearest side of the V-incision is connected with a pull-wire inserted into a longitudinal channel in the tube wall, said pull-wire being connected with a ring positioned outside the tube.

After a preceding estimate of the lumen in the two sides of nasal fossae by tentatively inserting the tube, the operator chooses the most spacious side allowing the tube to be inserted with its cylindrical side against nasal septum so that the bevelled end of the tube, the patient's head being placed in normal-anatomic position (the nose turning right up and the neck resting directly on the bed without any intermediate layer), after passage through choana (posterior nares) points towards the plane of glottis. Thus, in order to avoid manufacturing a right and a left model of the tube of the above type, it is advantageous according to the invention that the tube be provided with two mainly V-shaped, preferably symmetrically opposed incisions in two opposite sides of the tube and in relation to a plane (saggital plane; containing the longitudinal axis of the tube, said two incisions being preferably equally spaced from the distal end, either incision being closed by an associated, bellows-shaped membrane, and in that there are provided two associated pull-wires inserted in their respective, longitudinal channel of the tube wall, with pertaining rings.

With a view to avoid manufacturing a right and a left model in respect of the frequently considerable difference in lumen between the two sides of nasal fossae, another solution aims at making the tube tip manually pivotal about a luminal axis in conformity with the actual anatomic configuration. The connection between the tube tip and the tube is inseparable with a rotational angle of 180° and includes locking means ensuring that the bevelled edge of the tube tip in both positions is on the plane of glottis.

Both designs allow for inserting the tube tip with its cylindrical wall facing nasal septum and later on, during the intubation course, for graduating the curvature of the tube tip via pull means.

At the initiation of tensioning, the end piece is at first made to tilt more or less in relation to the tube because of the difference in flexural rigidity. If after the maximum deflection of the end piece a pulling force is still exerted on the pull means, the the tube proper is bent to increase its curvature.

In other words, instead of a curvature uniformly effected throughout the length of the tube and which the operator may redistribute in response to the anatomic-configuration, the tube according to the invention provides for obtaining a selective, graduated change of curvature of the distal end (tube tip) based on the auscultorically determined position thereof in upper tracheal passage and in relation to aditus oesophagus.

The intubation of trachea is normally effected by exposure of aditus laryngis when the patient is in supine position but in case of heavily injured patients, the nasal intubation with the tube according to the invention may be applied as well in the side position, the so-called NATO-position, and in abdominal position, inter alia because the operator after having preliminarily adjusted the position of the end piece only needs one hand for the insertion of the tube by suitable two-phased change of the curvature of the tube tip while the free hand simultaneously adjusts and supports the patient's head.

The opening angle of the V-shaped incision(s) may for instance amount to 60° so that the pivot angle of the end piece in the initiating phase of intubation may amount to about 60° to either side in the saggital plane (corresponding to the drawing plane) about an axis perpendicular thereto in coincidence with the peak of the V-shaped incision which acts as a hinge for the tilting of the end piece. This zone, at the peak of the V-shaped incision, of the tube wall acts as a hinge for the tilting of the end piece. The bellows membrane ensures the necessary, tight separation between the interior of the tube and the environments in nasal fossae, pharynx and aditus laryngis of the patient.

The invention will now be explained in more detail with reference to the schematical drawings, in which

Fig. 1 illustrates an embodiment of an endotracheal tube according to the invention,

Fig. 2 is the front end of the tube with the end piece turned 90° in relation to the drawing plane of Fig. 1,

Fig. 3, on a larger scale, a section through the tube end with an example of designing the coupling between the end piece and the tube proper,

Fig. 4 is an example of designing an extension to be connected at the proximal end of the tube, and

Fig 5 a second embodiment of the tube according to the invention.

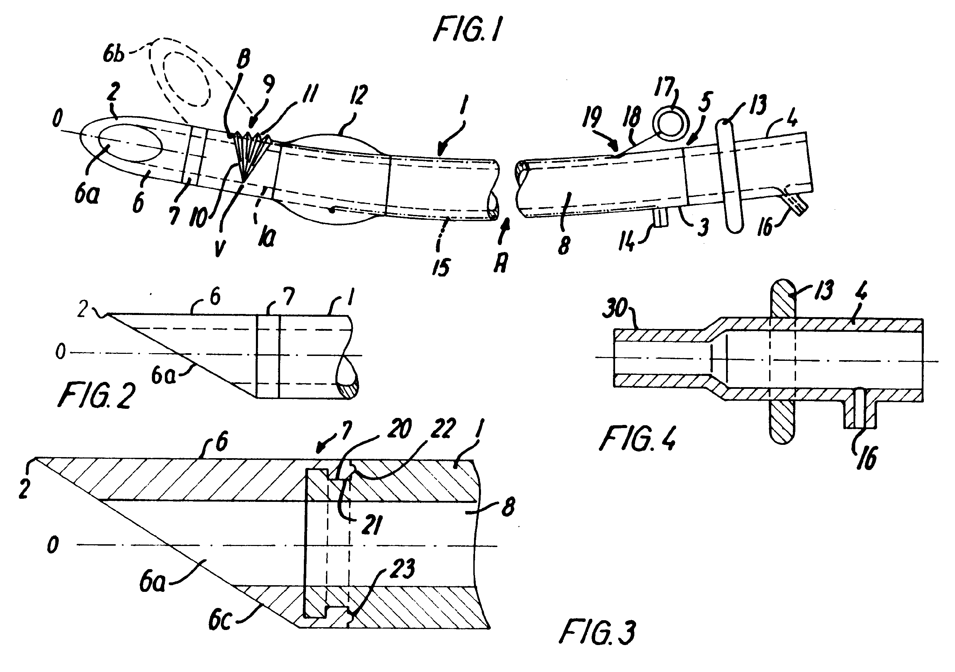

Fig. 1 illustrates an example of designing an endotracheal tube according to the invention.

In the following description it is supposed that nasal intubation is effected, but oral intubation may be effected as well, mainly in the same manner.

The tube generally designated 1 is made from resilient plastic material and is slightly curved as shown in Fig. 1 (in the drawing plane of Fig. 1). In view of the fact that this tube is comparatively long (e.g. about 30 cm) compared to the diameter (e.g. about 1.5 cm) the tube is for the sake of clearness cut off at A in Fig. 1, but it is obvious that the two parts of the tube actually are integral.

Reference numeral 2 shows the distal end of tube 1 which by a doctor, a surgeon or an anaesthesiologist, hereinafter named operator, is to be inserted in nasal fossae of a patient and onwards to hypopharynx. An extension 4 is coupled to the proximal end 3 of tube 1 and is operated by the operator during intubation. As it will be explained later on, extension 4 may be disconnected tube 1, the joint between said two members being shown by reference numeral 5. The external surface of tube 1 is provided with a centimetre-indication (not shown) used by the operator during intubation.

Tube 1 is at distal end 2 provided with an end piece 6 which is connected by a coupling 7 pivotal about its longitudinal axis 0 with tube 1 proper. The lumen 8 in tube 1 extends all the way from proximal end 3 to distal end 2 where it discharges into the opening 6a in end piece 6. 1a designates the internal surface of tube 1.

As shown by 9 a mainly V-shaped incision 10 from the convex side of the tube to a point V at the internal surface la of the tube at the concave side of the tube is provided in tube 1 in the vicinity of coupling 7. A bellows-shaped membrane of plastic or elastomeric material is shown by 11. At a short distance from bellows 11 tube 1 is provided with an inflatable sleeve 12, a so-called cuff.

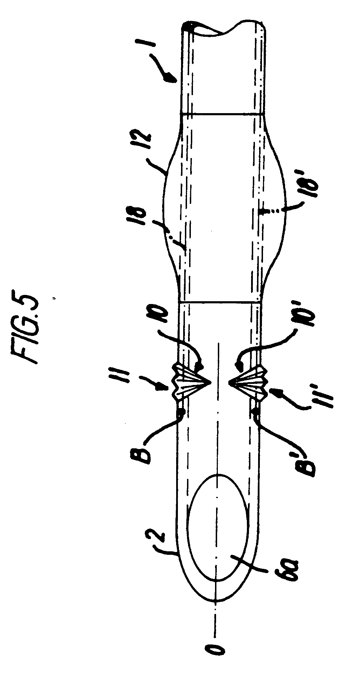

In the vicinity of proximal end 3 extension 4 is provided with a solid collar 13 (see also Fig. 4) by which the operator with his thumb and index finger can hold tube 1. 14 designates a fitting or connector for connection with a thin tube with valve means for the supply of air to cuff 12 through a longitudinal channel 15 (illustrated in double dot-and-dash line) extending in the tube wall and along the convex side of the tube from connector 14 to the interior of cuff 12.

16 is a connector to be connected with a stethoscope, not shown. 17 is an ring to be activated by the operator's index finger. Said ring 17 is connected with a pull-wire 18 of metal which as illustrated in a dotted line in Fig. 1 is passed through a channel extending along the concave side of the tube and onwards to a point B of the tube wall on the side facing distal end 2 in relation to V-incision 10. Pull-wire 18 leaves its longitudinal channel at a point 19 at a suitable distance from collar 13.

6b in Fig. 1 illustrates (in dotted line) the position of end piece 6 when the operator by tensioning the ring 17 and thus also pull-wire 18 has made the end piece to tilt an angle substantially equal to the opening angle of V-incision 10, while bellows membrane 11 is collapsing.

As previously mentioned, end piece 6 is by means of coupling 7 pivotal about its axis corresponding to the longitudinal axis of the tube. Fig. 2 illustrates the proximal end of the tube with end piece 6 turned 90° in relation to the plane in Fig. 1 and, as it will appear from Figs 2 and 3, end piece 6 is bevelled in relation to axis 0.

Fig. 3 illustrates on a larger scale and axially the proximal end of the tube.

The reason why end piece 6 is pivotal about its axis will be explained later on. Fig. 3 is a design example of how to obtain such a pivotability.

At the end facing tube 1 end piece 6 is provided with a radially inwards extending collar 20 for insertion into a corresponding groove 21 at the end of tube 1 so that end piece 6 in the axial direction is kept firmly to the tube - which is of utmost importance with respect to safety, since end piece 6 during intubation must never be released from tube 1 - but, nevertheless, so that it may be pivoted about its longitudinal axis 0. Two small locking projections 22, 23, serve by engagement with corresponding depressions to lock end piece 6 with tube 1 in determined, 180° separate angle positions. Such locking projections 22, 23, however, do not need to be present, if the designing of collar 20 and the corresponding groove 21 makes provision for such a dimensioning as to obtain a comparatively tight sliding fit so that the end piece 6 brought into a specific angle position by the operator occupies this position during intubation.

In the illustrated design example end piece 6 is connected directly with tube 1, but in view of the fact that the tube is flexible per se and has a wall of relatively small wall thickness it may be advantageous to connect end piece 6 with an intermediate piece, not shown in the drawings, which is in firm and permanent communication with tube 1 proper and may be made from a suitable plastic material of sufficient thickness for obtaining a satisfactory design of the individual locking parts of the coupling.

The mode of effect of this endotracheal tube will now be explained in more detail.

A rhinoscopic estimate of the nasal fossae lumen, particularly with a view to reveal possible skewness in nasal septum, may for instance be effected prior to intubation.

The tube tip, i.e. end piece 6 may be pivoted manually in relation to tube 1 so as to be inserted into the most spacious half of nasal fossae with its cylindrical side facing nasal septum and thus with the bevelled side facing the wing of the nose.

Tube 1 with end piece 6 pre-adjusted in angle position is rubbed into an anaesthetic creme and is inserted while in connection with extension 4 and the stetoscope, until being resisted by the rear wall of nasopharynx. Tube 1 is pulled a little back and upon tensioning pull-wire 18 by means of ring 17 the tube tip is made to pivot towards the axis of pharynx between the rear wall and soft palate of nasopharynx, thereby preventing injury to the mucous membrane of the rear wall at the risk that the end edge of end piece 6 penetrates under the mucous membrane.

Due to its slight curvature tube 1 bears against the rear wall of pharynx and is inserted further while the operator through the stetoscope ascertains spontaneous respiratory sound.

While further inserting tube 1 the respiratory sound suddenly ceases as a sign that the tube tip has passed the oesophageal entry.

Tube 1 is now pulled slowly back until the respiratory sound occurs again as a sign that the tube tip is now located opposite the lowermost limit of aditus laryngis. Simultaneously with pulling back, a slight intensifying activation of the wire drive is effected. When the slight resistance ceases tube tip is positioned at aditus laryngis and the tube may now under stetoscopic control of continued respiration be further inserted by slackening the pull on ring 17.

Owing to the preliminary adjustment of end piece 6, as described, with the bevelled edge in the plane of Fig. 1 which, as mentioned, promotes the insertion into the nasal fossae, said edge will aim at the plane of glottis and so pass without inducing unnecessary traumatism.

After the passage of glottis a reflex cough is elicited and the tube is further inserted some centimetre (read on the external side of tube 1 in relation to nostril), following which a suitable filling of cuff 12 is effected by supplying air through connector 14 and the associated channel 15. The correct placing under glottis is ascertained by resistance against attempts of pulling back.

Extension 4 is then removed and proximal end 3 of tube 1 is connected with the anaesthetic source.

Fig. 4 is an example of designing extension 4 made from solid plastic material and provided with a fixed collar 13, connector hose 16 to be connected with a stethoscope and with stepped profiled cone 30 with a view to be connected with tubes of different lumen 30 at the proximal end of tube, cf. Fig. 1.

Fig. 5 illustrates a second embodiment of the part of the tube shown to the left in Fig. 1 (to the left of cut A in the drawing of Fig. 1).

Fig. 5 shows again the distal end 2 of tube 1, orifice 6a provided therein in an oblique plane in relation to the drawing plane of Fig. 5, and cuff 12.

In the embodiment illustrated in Fig. 5 tube 1 now extends straight, i.e. without the curvature traditional of intubation tubes and, moreover, distal end 2 of the tube, contrary to the embodiment according to Fig. 1, is designed without end piece 6 and associated coupling 7.

In order to make it possible for the operator in dependence on nasal fossae lumen to use the same tube for arbitrary insertion into the left or right nostril, tube 1 now includes between distal end 2 and cuff 12 two, substantially V-shaped incisions 10, 10' preferably provided in two opposite sides of the tube, symmetrically opposed and in relation to the saggital plane (plane including the longitudinal axis 0 of the tube and in the plane of Fig. 5).

Said two V-shaped incisions - each with an opening angle likewise preferably of 60° - are also closed by respective bellows-shaped membranes 11, 11' and are preferably positioned at equal distance from the distal end of the tube.

In longitudinal, diametrically opposed channels of the tube wall there are inserted associated pullwires 18, 18' of the same type as described above, with associated rings, not shown in Fig. 5, said pull-wires 18, 18' secured in diametrically opposed points B, B' - in the same manner as explained in the preceding - making it possible to the operator to tilt the distal end in the one or other direction relative to the saggital plane.

The embodiment according to Fig. 5 offers the advantage already mentioned that it is possible with the same tube to effect right-sided or left-sided intubation and it entails, moreover, the advantage that there is no need of designing the tube with pivotal end piece (simpler manufacturing, safety against accidental separation of end piece from the tube) and that the resiliency of the material (plastic) of the tube wall between the peaks of the two V-incisions causes the tube during insertion into nasopharynx with patient's head in normal-anatomic position, to bear automatically against the rearmost pharynx wall, thereby for certain engaging the entry of oesophagus with cessation of the ausculatorily ascertained spontaneous respiratory sound that is the important reference of the two-phased intubation course.

In order to ensure that the course of the two-phased intubation manoeuvre be effected as quietly and relaxed as possible, the extension may be provided with some sort of pistol butt having a slide device and ring for activating the metallic wire.

Finally, it is emphasized that a pull-wire of metal is used, but it is obvious that it falls within the scope of the invention to make use of a pull device designed in any other suitable way.

## Claims

1. An endotracheal tube for blind, nasal intubation and of the type in which the tube (1) comprises a distal end (2), a proximal end (3), an inflatable cuff (12) positioned at a relatively short distance from the distal end (2), said cuff having associated channels (15) extending longitudinally in the wall of tube (1) and intended to connect the interior of the cuff (12) with an air supply hose (14) provided with valve and pull means (17, 18) for altering the curvature of the tip of the tube (1) during intubation, characterized in that the tube (1) between the distal end (2) and the cuff (12) presents a smaller flexural rigidity than the remainder of the tube.

2. An endotracheal tube as claimed in claim 1, characterized in that said smaller flexural rigidity is provided in that the wall of the tube (1) includes at least one, mainly V-shaped incision (10), that a flexible bellows-shaped membrane (11) is provided for air-, gas- and liquidtight closing of the incision (10), and that a point (B) of the tube wall, between the distal end (2) and the nearest side of the V-incision is connected with a pull-wire (18) inserted into a longitudinal channel in the tube wall, said pull-wire being connected with a ring (17) positioned outside the tube (1).

3. A tube as claimed in claim 2, characterized in that the tube is provided with two mainly V-shaped, preferably symmetrically opposed incisions (10, 10') in two opposite sides of the tube and in relation to a plane (saggital-plane) including the longitudinal axis of the tube, said two incisions (10, 10') being preferably equally spaced from the distal end (2), that either incision (10, 10') is closed by an associated, bellows-shaped membrane (11), and in that there are provided two associated pull-wires, with pertaining rings, inserted in their respective, longitudinal channel in the tube wall.

4. A tube as claimed in claim 3, characterized in that the two incisions (10, 10') have such an opening angle that the tube tip may be moved an angle of preferably ±60° in relation to said saggital plane.

5. A tube as claimed in any of the preceding claims, characterized in that an end piece (6) pivotal about the longitudinal axis of the tube is provided at the distal end of the tube, said end piece being provided with said opening (6a) and inseparably connected with the tube (1) by means of a pivot coupling (7).

6. An endotracheal tube as claimed in any of the preceding claims, characterized in that it is intended to be connected with an extension (4) made from plastic material and provided eith a stepped profiled cone (30) to be connected with tubes of different lumen, with a leverage device having a collar against which fingers are bearing upon ring activation of the tube tip (6) via the metallic wire (18), and with a connector hose (16) for a stethoscope.

**Patentansprüche**

1. Luftröhrenrohr für blinde, nasale Intubation und von der Art, wo das Rohr (1) ein distales Ende (2), ein proximales Ende (3) und eine in verhältnismässig kleinem Abstand vom distalen Ende (2) angeordnete aufblasbare Manschette (12) umfasst, welche Manschette (12) zugehörige Kanäle (15) aufweist, die in Längsrichtung in der Rohrwand (1) verlaufen und dazu vorgesehen sind das Innere der Manschette (12) mit einem mit Ventil und Zugmitteln (17, 18) versehenen Luftzufuhrschlauch (14) zu verbinden, um während der Intubation die Krümmung der Rohrspitze (1) zu ändern, dadurch gekennzeichnet, dass das Rohr zwischen dem distalen Ende (2) und der Manschette (12) eine geringere Biegesteifheit aufweist als der übrige Teil des Rohres.

2. Luftröhrenrohr nach Anspruch 1, dadurch gekennzeichnet, dass die erwähnte geringere Biegesteifheit dadurch entsteht, dass in der Rohrwand (1) mindestens ein vorwiegend V-förmiger Einschnitt (10) vorgesehen ist, dass zum luft-, gas- und flüssigkeitsdichtem Verschliessen des Einschnittes (10) eine elastische, balgförmige Membran (11) vorhanden ist, und dass ein zwischen dem distalen Ende (2) und der am nächsten liegenden Seite des V-Einschnittes (10) befindlicher Punkt (B) der Rohrwand mit einer in einen längsverlaufenden Kanal in der Rohrwand eingeführten Zugschnur (18) verbunden ist, welche Zugschnur mit einer ausserhalb des Rohres (1) angebrachten Öse (17) in Verbindung steht.

3. Rohr nach Anspruch 2, dadurch gekennzeichnet, dass das Rohr mit zwei vorwiegend V-förmigen, vorzugsweise symmetrisch gegenüberliegenden Einschnitten (10, 10') auf zwei entgegengesetzten Seiten des Rohres und im Verhältnis zu einer Ebene (Saggitalebene), die die Längsachse des Rohres einschliesst, ausgestattet ist, wobei sich die zwei Einschnitte (10, 10') vorzugsweise im gleichen Abstand vom distalen Ende (2) befinden, dass jeder Einschnitt (10, 10') von einer zugehörigen balgförmigen Membran (11) verschlossen ist, und dass zwei dazugehörende Zugschnüre mit entsprechenden Ösen, jede in ihren betreffenden längsverlaufenden Kanal in der Rohrwand eingesetzt, vorgesehen sind.

4. Rohr nach Anspruch 3, dadurch gekennzeichnet, dass die zwei Einschnitte (10, 10') einen solchen Öffnungswinkel aufweisen, sodass die Rohrspitze in einem Winkel von vorzugsweise ±60° im Verhältnis zu erwähnter Saggitalebene bewegt werden kann.

5. Rohr nach einem der vorhergehenden Ansprüche, dadurch gekennzeichnet, dass an dem distalen Ende des Rohres ein um die Längsachse des Rohres drehbares Endstück (6) vorgesehen ist, das die Öffnung (6a) aufweist und mittels einer Drehkupplung (7) mit dem Rohr (1) untrennbar verbunden ist.

6. Luftröhrenrohr nach einem der vorhergehenden Ansprüche, dadurch gekennzeichnet, dass es zum Zusammenkoppeln mit einem Verlängerstück (4) bestehend aus Kunststoffmaterial und mit treppenförmigen profilierten Konus (30) zum Anschluss an Rohre mit unterschiedlichem Lumen und mit einer Hebelvorrichtung mit einem Kragen zur Stütze von Fingern während der Ösenaktivierung der Rohrspitze (6) mittels des Metalldrahts (18) und mit einem Verbindungsschlauch (16) für ein Stethoskop vorgesehen ist.

**Revendications**

1. Tube endotrachéal pour intubation nasale à l'aveuglette et du type dans lequel le tube (1) comprend une extrémité distale (2), une extrémité proximale (3), une manchette gonflable (12) située à relativement courte distance de l'extrémité distale (2), ladite manchette étant associée à des canaux (15) qui s'étendent longitudinalement dans la paroi du tube (1) et qui sont prévus pour relier l'intérieur de la manchette (12) à un ajutage (14) d'alimentation en air avec soupape, et des moyens de traction (17, 18) pour modifier la courbure de l'extrémité du tube (1) durant l'intubation, caractérisé en ce que le tube (1), entre l'extrémité distale (2) et la manchette (12), présente une rigidité à la flexion plus faible que le reste du tube.

2. Tube endotrachéal selon la revendication 1, caractérisé en ce que ladite rigidité plus faible est obtenue par le fait que la paroi du tube (1) présente au moins une incision (10) essentiellement en forme de V, qu'une membrane (11) en forme de soufflet, étanche à l'air, aux gaz et aux liquides, clôt cette incision, et qu'un point (B)de la paroi du tube, entre l'extrémité distale (2) et le coté le plus proche de l'incision en V, est relié à un cordon de traction (18) logé dans un canal longitudinal dans la paroi du tube, ledit cordon de traction étant relié à un anneau (17) situé extérieurement au tube (1).

3. Tube selon la revendication 2, caractérisé en ce que le tube est pourvu de deux incisions (10, 10') essentiellement en forme de V, de préférence symétriquement opposées sur deux cotés opposés du tube et par rapport à un plan (plan sagittal) comprenant l'axe longitudinal du tube, lesdites deux incisions (10, 10') étant, de préférence, également espacées depuis l'extrémité distale (2), en ce que chaque incision (10, 10') est close par une membrane correspondante (11) en forme de soufflet, et en ce qu'il y a deux cordons associés de traction avec anneaux correspondants, logés dans leurs canaux longitudinaux respectifs dans la paroi du tube.

4. Tube selon la revendication 3, caractérisé en ce que les deux incisions (10, 10') ont un angle d'ouverture tel que l'extrémité du tube peut être déplacée d'un angle de préférence ± 60° par rapport au plan sagittal.

5. Tube selon l'une quelconque des revendications précédentes, caractérisé en ce qu'une pièce d'extrémité (6), pivotable autour de l'axe longitudinal du tube, est prévue à l'extrémité distale du tube, ladite pièce d'extrémité étant pourvue d'une ouverture (6a) et étant reliée, de façon inséparable, avec le tube à l'aide d'un accouplement à pivotement (7).

6. Tube endotrachéal selon l'une quelconque des revendications précédentes, caractérisé en ce qu'il est prévu pour être relié à un prolongement (4) constitué à partir d'un matériau plastique et pourvu d'un cône profilé en marche (30) pour le branchement de tubes de lumière différente et d'un moyen d'appui avec un collier permettant l'appui des doigts lors de la commande, par anneau, de l'extrémité (6) du tube à l'aide du cordon métallique (18), ainsi que d'un ajutage (16) de branchement d'un stéthoscope.

FIG.1

FIG.2

FIG.3

FIG.4

EP 0 429 523 B1

FIG.5